# EUROPEAN PATENT APPLICATION

(11) **EP 2 363 070 A1**
(43) Date of publication of application: **07.09.2011**
(21) Application number: 10170980.6
(22) Date of filing: 25.07.2005
(51) Int. Cl.: A61B 8/00, A61B 10/00

(54) **Ultrasonic puncture needle**

(62) Divisional of application: 05766216.5
(71) Applicant: Hakko Co., Ltd., Nagano-ken (JP)
(72) Inventor: Maruyama, Masaru, Nagano 389-0806 (JP); Kitagawa, Shiro, Tokyo 133-0033 (JP); Komatsu, Masayoshi, Sendai-shi Miyagi 983-0852 (JP)
(74) Representative: Gill, David Alan

(57) **Abstract**

A puncture needle capable of clear imaging even if an angle to the irradiation direction of a detection wave such as an ultrasonic wave of an ultrasonic probe is small. According to one embodiment, a ultrasonic puncture needle (1) that conducts puncturing while observing a puncturing condition by means of a ultrasonic image is formed with an edge (13) at the tip end of a puncture needle main body (11), and formed at a specified position of the puncture needle main body (11) with at least one corner cube mirror (12) formed by a triangular pyramid recess. Since a ultrasonic wave oscillated from the ultrasonic probe is reflected off the corner cube mirror (12) to be returned to the ultrasonic probe, the reflected wave progresses in the same direction as the incident direction of the ultrasonic wave to be positively returned to the probe even when an angle formed by the irradiation route of a ultrasonic wave and the axis of the puncture needle is small.

## Description

The present invention relates to a puncture needle for a medical application, and more particularly, to a puncture needle used under displaying ultrasonic image.

In the medical field, a puncture needle (biopsy needle, insulated electrode needle) is used, for example, for gathering a part of a tissue or a cell of a human organism is gathered for the purpose of diagnostic confirmation of a lesion, or for precisely finding a location of a nerve. For this case, an ultrasonic diagnostic equipment is used from a requirement of precisely recognizing a location of the puncture needle, particularly a location of tip end thereof. For example, in a case of using a biopsy needle, needle puncturing is conducted while observing an ultrasonic echo from the tip end of the puncture needle by displaying a pictorial image, to precisely finding as to whether a tissue collecting part of the tip end of the puncture needle (a needle point or a notch) reaches the lesion. Further, this biopsy needle often has a dual structure comprising an inner tube and an outer tube.

On the other hand, another puncture needle called as an insulated electrode block needle in general has a single tube structure (single-needle), in that an electrical insulation coating is provided on a surface of the needle tube except an edge surface thereof. The insulated electrode block needle is generally used for searching the nerve by using a muscle contraction caused by an electric stimulation, in order to conduct a nerve block by dosing anesthetics or painkiller to the nerve. In recent years, a manual technique to puncture the needle while observing the ultrasonic image together with energization by a conventional nerve stimulator (puncturing under irradiation of the ultrasonic wave and confirmation by the nerve stimulator) has been widely used in an approach of the nerve search.

The ultrasonic image is an image obtained by irradiating an ultrasonic wave from an ultrasonic probe (ultrasonic transducer) as a transmitting and receiving device of the ultrasonic wave to the puncture needle via a medium, receiving a reflected wave (ultrasonic echo) from the puncture needle at the ultrasonic probe, and conducting an image processing on the reflected wave to display an image on a display unit.

As the conventional puncture needles to be used while displaying the ultrasonic image, for example, a puncture needle provided with a protrusion, a V-groove or the like at its outer periphery surface of the tip end (for example, see patent document 1), a puncture needle provided with a circular groove at its outer periphery surface (for example, see patent documents **2, 3**), a puncture needle provided with an enhanced portion comprising a cylindrical protruding portion and a circular groove at its tip end (for example, see patent documents **4, 5**) are known. Configuration of the protrusion, the groove and the like provided at the tip end of any of these puncture needles is designed for easily generating a reflection of the ultrasonic wave.
Patent document **1**: Japanese Patent Laid-Open No. 3-228748
Patent document **2**: Japanese Patent Laid-Open No. 11-76254
Patent document **3**: Japanese Utility Model Laid-Open No. 3-73113
Patent document **4**: Japanese Patent Laid-Open No. 2004-181095
Patent document **5**: Japanese Patent Laid-Open No. 2003-144436

However, according to the conventional puncture needles in which the ultrasonic wave is reflected back by machining the tip end, an angle made by an irradiation path of the ultrasonic wave and an axis of the puncture needle is small. In particular, when the angle is not great than **45**°, a level of the ultrasonic echo returned to the ultrasonic probe is decreased, so that the ultrasonic image of the puncture needle becomes unclear, and as a result, it is difficult to recognize a precise location of the puncture needle.

Accordingly, it is an object of the invention to provide a puncture needle, which can be clearly imaged, even though an angle made by an irradiation angle of a detection wave such as an ultrasonic wave of an ultrasonic probe and an axis of the puncture needle is small.

According to a feature of the present invention, so as to solve the above problem, an ultrasonic puncture needle for conducting puncturing while observing a puncturing condition by an ultrasonic image, comprising÷ a needle tube main body comprising an edge at its tip end characterized in that: at least one corner cube mirror configured by three mirror surfaces that are perpendicular to each other is provided at a predetermined position of the needle tube main body. Herein, the needle tube main body' may comprise an inner needle or an outer needle of a double needle, a single tube structure, or other kinds of needles. The corner cube mirror is provided at an outer surface or an edge surface thereof, regardless the needle tube main body comprises the inner needle, the outer needle, or the single tube structure.

### [Effects of the Invention]

According to the puncture needle of the present invention, it is possible to clearly image a needle tube main body, particular a tip end of the needle tube main body, even though an angle made by an irradiation angle of a detection wave such as an ultrasonic wave of an ultrasonic probe and an axis of the puncture needle is small.
**FIG.1** shows a puncture needle in a preferred embodiment according to the present invention, wherein **FIG.1A** is a perspective view thereof, and FIG.1B is a cross sectional view thereof along A-A line in **FIG.1A****;**
**FIG.2** shows a corner cube mirror shown in **FIG.1****,** wherein **FIG.2A** is an explanatory diagram showing a structure and a function of the corner cube mirror, and **FIG.2B** is an explanatory diagram showing an example of application; and
**FIG.3** shows a double needle provided with the corner cube mirror, wherein **FIG.3A** is a perspective view of a structure in which the corner cube mirror is provided at an edge surface of an inner needle, and **FIG.3B** is a perspective view of a structure in which the corner cube mirror is provided at an inner periphery surface of an outer needle.

### [Reference numerals]

- **1**: puncture needle
- **11**: puncture needle main body
- **12**: corner cube mirror
- **12**a, **12**b, **12**c: first to third mirror surfaces
- **13**: edge
- **14**: edge surface
- **20**: ultrasonic probe
- **21**: outer needle
- **22**: edge surface of the inner needle
- **24**: edge surface of the outer needle
- **30**: double needle

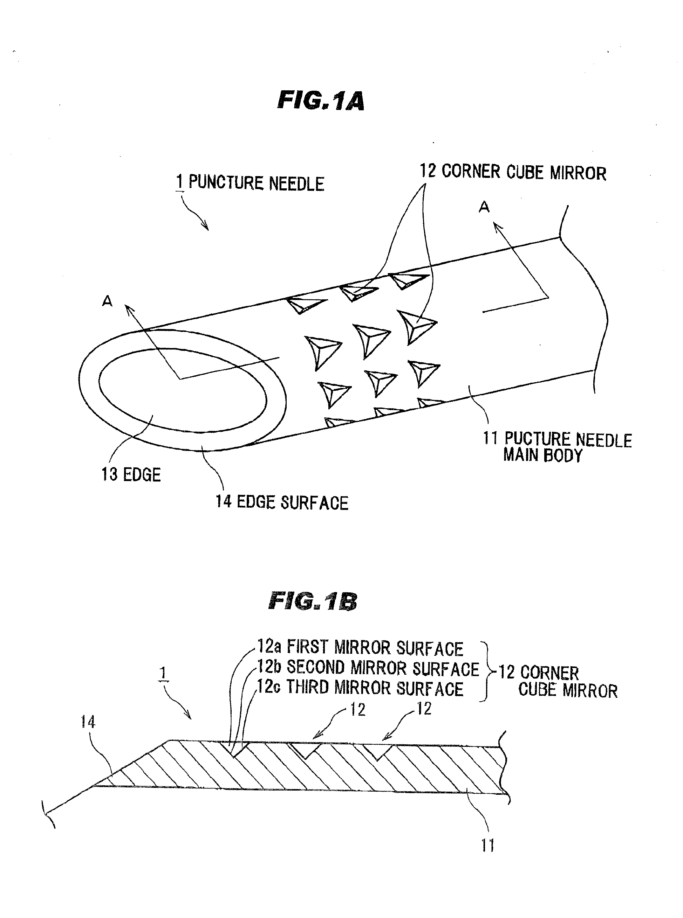

**FIG.1** shows a puncture needle in a preferred embodiment according to the present invention. In **FIG.1, FIG.1A** is a perspective view thereof, and **FIG.1B** is a cross sectional view thereof along A-A line in **FIG.1A**

An ultrasonic puncture needle (hereinafter referred as "puncture needle") **1**, which is a needle tube, comprises a puncture needle main body **11** which is a needle tube main body comprising a metal capillary made of stainless steel or the like, and a plurality of corner cube mirrors **12** provided at an outer surface (outer periphery surface) of a tip end of the puncture needle main body **11**.

The puncture needle main body **11** comprises an edge **13** including an edge surface 14 made by diagonally cutting its tip portion. The corner cube mirror **12** comprises a triangular pyramid-shaped recess provided at a surface of the puncture needle main body 11 at a region in vicinity of the edge **13**, and the corner cube mirrors **12** are formed along three lines with a predetermined interval in a circumferential direction of the puncture needle main body **11**. For example, the corner cube mirrors **12** are disposed with an interval of **45**° with respect to a center of the needle tube, and eight mirrors are formed per one line. In addition, the number of the corner cube mirrors **12** to be used and the number of lines are not limited to eight and three respectively as described above, and may be arbitrary numbers. The corner cube mirrors **12** are provided at the surface of the puncture needle main body **11**, however, the present invention is not limited thereto, and may be provided at an inner periphery surface of the puncture needle main body **11** or both of them.

It is preferable to form the corner cube mirror **12** in vicinity of the edge **13**, so as to clarify an imaging of the tip end. For example, the corner cube mirror **12** may be formed by electric discharge machining. Alternatively, the corner cube mirror **12** may be punched by a tool such as punches.

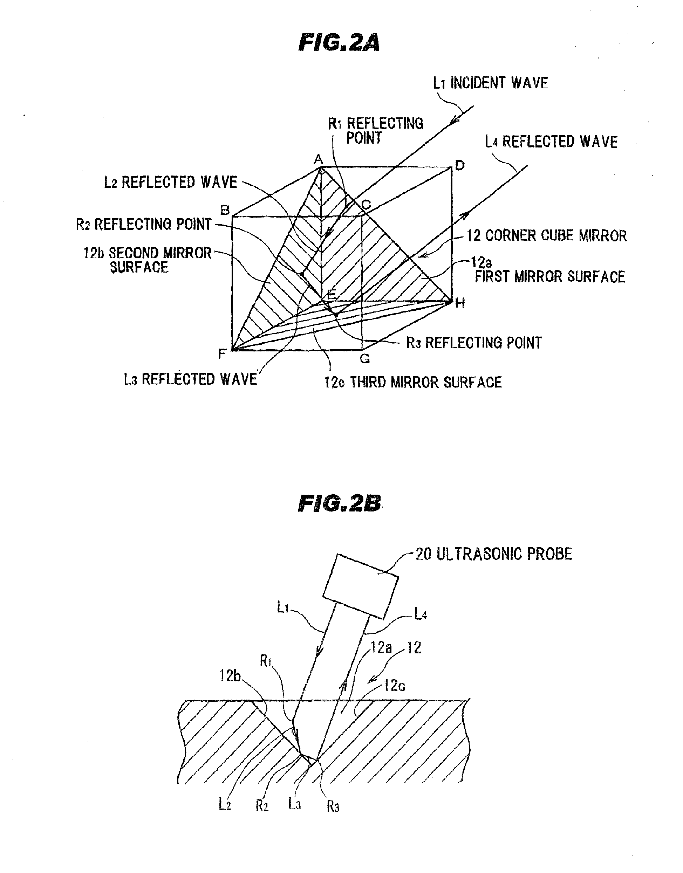

**FIG.2** shows a function of the corner cube mirror. This corner cube mirror **12** is configured by three mirror surfaces that are perpendicular to each other, and an incident wave is reflected back by the three mirror surfaces, and emitted as an output wave that is parallel to the incident wave.

As shown in **FIG.2A**, three faces (ABFE, EFGH, AEHD) of a cube (ABCD-EFGH) are provided as mirror surfaces, and AFH is opened. An opening with a triangular-pyramid shape having a base point E and a top face AFH is provided as the corner cube mirror **12**.

In **FIG.2A**, an incident wave L₁ which is incident to the corner cube mirror **12** is reflected at a reflecting point R₁ of a first mirror surface **12**a in the AEHD face to provide a reflected wave L₂, the reflected wave L₂ is reflected at a reflecting point R₂ of a second mirror surface **12**b in the ABFE face to provide a reflected wave L₃, and the reflected wave L₃ is reflected at a reflecting point R₃ of a third mirror surface **12**c in the EFGH face to provide an output wave L₄.

In **FIG.2B**, the incident wave L₁ is emitted from an ultrasonic probe 20, and the output wave L₄ is received by the ultrasonic probe **20**. According to this structure it is possible to detect the edge **13** including the edge surface **14** of the puncture needle main body **11** of the puncture needle **1** by virtue of the corner cube mirrors **12**.

As described above, the incident wave and the reflected wave of the ultrasonic wave which is incident to the corner cube mirrors **12** are parallel or substantially parallel to each other. Therefore, the ultrasonic wave transmitted from the ultrasonic probe **20** is necessarily reflected at the corner cube mirror **12** to be returned to the ultrasonic probe **20**, so that the reflected wave is directed to an output direction of the incident wave of the ultrasonic wave and surely returned to the ultrasonic probe **20** that is an emitting side of the ultrasonic wave, even when the angle made by the irradiation path of the ultrasonic wave and the axis of the puncture needle is small.

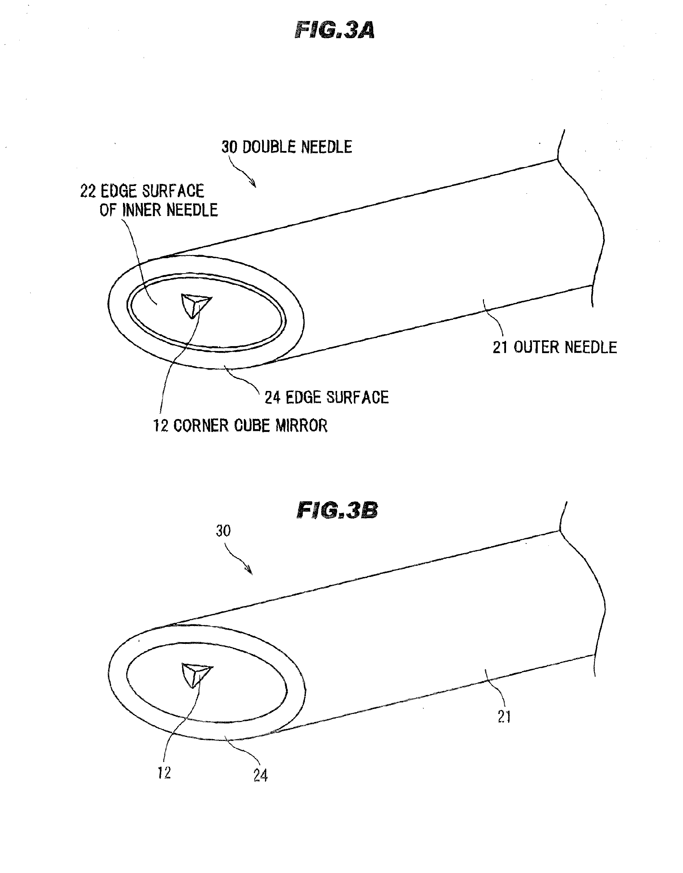

**FIG.3** shows a double needle **30** provided with the corner cube mirrors, wherein **FIG.3A** is a perspective view of the double needle **30** in which an inner needle (no reference numeral) having an edge surface **22** is inserted into an outer needle **21** having an edge surface **24**, and **FIG.3B** is a perspective view of the outer needle **21** from which the inner needle is detached.

In **FIG.3A**, the inner needle comprises the corner cube mirrors **12** provided on the edge surface **22**. According to this structure, it is possible to precisely detect a tip end of the double needle **30**.

In **FIG.3B**, the corner cube mirror **12** is provided on an inner periphery surface of the edge surface **24** of the outer needle **21**. According to this structure, it is possible to precisely detect a tip end of the outer needle **21** in the state that the inner needle is detached.

According to this preferred embodiment, following effects can be obtained.
(a) By providing the puncture needle with the corner cube mirror, the incident wave and the reflected wave of the ultrasonic wave in the puncture needle are made parallel or substantially parallel to each other, so that the ultrasonic wave output from the ultrasonic probe is reflected at the puncture needle and returned to the ultrasonic probe. Therefore, even though the angle made by the irradiation path of the ultrasonic wave and the axis of the puncture needle is reduced, the ultrasonic wave is reflected by the corner cube mirror and returned to the ultrasonic probe, so that it is possible to clearly image the puncture needle, particularly, the tip end thereof.
(b) By providing the corner cube mirror in vicinity of the edge, it is possible to clarify the imaging of the tip end.
(c) By providing a plurality of the corner cube mirrors at a periphery of the puncture needle, the incident wave and the reflected wave of the ultrasonic wave in the puncture needle are made parallel or substantially parallel to each other regardless the orientation of the puncture needle, so that it is possible to clearly image the puncture needle regardless the orientation of the puncture needle.
(d) By providing the corner cube mirrors in plural lines, it is possible to surely generate the reflected wave.
(e) Since the corner cube mirror has a good reflecting efficiency, so that it is possible to obtain a good reflecting property regardless the type of the puncture needle. In particular, by providing the corner cube mirror at the outer surface of the inner needle of the double needle, it was possible to obtain an excellent reflecting property by a synergistic effect with an air layer existing between the inner needle and the outer needle. However, it was also possible to obtain the excellent reflecting property according to a structure in which the corner cube mirror is provided at an outer surface of the single tube structure and a coating is provided at the outer periphery thereof.

The present invention is not limited to the preferred embodiment described above, and may be modified without going beyond or transforming a technical concept of the present invention.

For example, the present invention may be applied to all puncture needles to be punctured to a target while observing a location of the needle tube punctured into the human body by the ultrasonic echo, such as a PTC (Percutaneous Transhepatic Cholangiography) needle for cholangiography, a puncture needle for an ultrasonic endoscope, and the like.

In case that a fluorine resin is coated on a part other than the edge 13 in the puncture needle provided with the corner cube mirror **12**, in order to compose an insulated electrode block needle, it possible to clearly image the insulated electrode block needle, similarly to the above. By the way, since the conventional insulated electrode block needle does not comprise the corner cube mirror **12**, the image was unclear due to an attenuation caused by the insulation coating.

In addition, the present invention is not limited to the use of the ultrasonic wave, and may be applied, for example, to a light such as a laser beam, electron beam, or the like. Therefore, the present invention may be applied to an application for confirming a location of an article to be detected such as needle by using the reflected wave of an electromagnetic wave by the corner cube mirror. Further, the reflecting surface of the corner cube mirror **12** is not necessarily a plane surface.

The corner cube mirror formed at the outer surface of the needle tube can generate the reflected wave that is parallel or substantially parallel to the incident wave of the detection wave of the ultrasonic wave or the like, so that it is applicable to a purpose for clarifying the image of the needle tube existing in a range to which the detection wave of the ultrasonic wave or the like output from the ultrasonic probe or the like reaches, for example, a medical apparatus and a medical equipment.

## Claims

1. An ultrasonic puncture needle for conducting puncturing while observing a puncturing condition by an ultrasonic image, comprising:
a needle tube main body comprising an edge at its tip end; and
at least one mirror portion provided at a predetermined position of the needle tube main body,
**characterized in that** the mirror portion comprises a first mirror surface, a second mirror surface and a third mirror surface, each of which has a reflecting direction of a detection wave different from each other, the first to third mirror surfaces are located to be adjacent to each other, and the first to third mirror surfaces have a base point E as a common point.

2. The ultrasonic puncture needle, according to Claim 1, wherein the mirror portion comprises a triangular-pyramid shape.

3. The ultrasonic puncture needle, according to Claim 1, wherein an apex of an opening of the mirror portion and the base point E are formed along a straight line parallel to a pipe axis.

4. The ultrasonic puncture needle, according to Claim 1, wherein the mirror portion receives the detection wave and reflects a reflected wave which is parallel or substantially parallel to the detection wave.

5. The ultrasonic puncture needle, according to Claim 1, wherein the first mirror surface receives the detection wave and reflects a first reflected wave, and the third mirror surface receives the first reflected wave via the second mirror surface and reflects a second reflected wave that is parallel or substantially parallel to the detection wave.

6. The ultrasonic puncture needle, according to Claim 5, wherein second reflected wave is directed to an output direction of the detection wave.

7. The ultrasonic puncture needle, according to Clam 1, wherein the mirror portion is provided at an outer surface of the needle tube main body.

8. The ultrasonic puncture needle, according to Claim 1, wherein the mirror portion is provided at an edge surface of the needle tube main body.

9. The ultrasonic puncture needle, according to Claim 7, wherein a plurality of mirror portions are formed in plural lines in a longitudinal direction of the outer surface of the needle tube main body.

10. The ultrasonic puncture needle, according to Claim 1, wherein the needle tube main body comprises a single tube structure, an inner needle of a double needle or an outer needle of the double needle.

11. The ultrasonic puncture needle, according to Claim 10, wherein the mirror portion is provided at an inner surface of an edge of the outer needle.

12. The ultrasonic puncture needle, according to Claim 1, wherein the needle tube main body is provided with a resin coating at a part other than the edge.

13. The ultrasonic puncture needle, according to Claim 1, wherein the mirror portion is formed by electric discharge machining.

14. The ultrasonic puncture needle, according to Claim 1, wherein the mirror portion is punched.

15. The ultrasonic puncture needle, according to Claim 1, wherein at least one of the first to third mirror surfaces is not a plane surface.

16. An ultrasonic puncture needle for conducting puncturing while observing a puncturing condition by an ultrasonic image, comprising:
a needle tube main body comprising an edge at its tip end; and
at least one corner cube mirror provided at a predetermined position of the needle tube main body.

17. The ultrasonic puncture needle, according to Clam 16, wherein the corner cube mirror is provided at an outer surface of the needle tube main body.

18. The ultrasonic puncture needle, according to Claim 16 or 17, wherein the corner cube mirror is provided at an edge surface of the needle tube main body.

19. The ultrasonic puncture needle, according to Claim 17, wherein a plurality of the corner cube mirrors are formed in plural lines in a longitudinal direction of the outer surface of the needle tube main body.

20. The ultrasonic puncture needle, according to any one of Claims 16 to 19, wherein the needle tube main body comprises a single tube structure, an inner needle of a double needle or an outer needle of the double needle.

21. The ultrasonic puncture needle, according to Claim 20, wherein the corner cube mirror is provided at an inner surface of an edge of the outer needle.

22. The ultrasonic puncture needle, according to Claim 16 or 21, wherein the needle tube main body is provided with a resin coating at a part other than the edge.

23. A puncture needle comprising a detection hole for reflecting a detection wave, wherein the detection hole comprises a first reflecting surface for receiving the detection wave from outside the puncture needle and reflecting a first reflected wave, and a third reflecting surface for receiving the first reflected wave via a second reflecting surface and reflecting a second reflected wave that is parallel or substantially parallel to the detection wave.
